# EUROPEAN PATENT APPLICATION

(11) **EP 2 642 259 A1**
(43) Date of publication of application: **25.09.2013**
(21) Application number: 13157378.4
(22) Date of filing: 01.03.2013
(51) Int. Cl.: G01F 23/04, H04Q 9/00

(54) **Sensor device and method for communicating with sensor devices**

(30) Priority: 22.03.2012 GB 201205074
(71) Applicant: AIRBUS OPERATIONS LIMITED, Filton Bristol Bristol BS99 7AR (GB)
(72) Inventor: Tichborne, Franklin, BRISTOL, BRISTOL BS99 7AR (GB); Lam, Joseph K-W, BRISTOL, BRISTOL BS99 7AR (GB); Frost, Mary, BRISTOL, BRISTOL BS99 7AR (GB); Haskins, Richard, BRISTOL, BRISTOL BS99 7AR (GB)
(74) Representative: Scott, Alistair Francis

(57) **Abstract**

A sensor device for a fuel system, the sensor device comprising sensing means configured to sense a property of fuel within the fuel system, and transmitting means configured to wirelessly transmit a signal representative of the sensed fuel property to a remote receiver. Also a sensor device comprising a photo-diode which is operable to function as an antenna or part of an antenna for the device. Also a method for communicating with a plurality of sensor devices.

## Description

### FIELD OF THE INVENTION

The invention relates to a sensor device, a sensor system comprising one or more of the sensor devices, a fuel system comprising the sensor system, an aircraft comprising the fuel system, and a method for communicating with a plurality of sensor devices.

### BACKGROUND OF THE INVENTION

An aircraft fuel system may comprise a plurality of sensors for sensing various properties of fuel in the fuel system. These sensors may be connected to a wiring harness, and the wiring harness may connect the sensors to a central processor. The wiring harness typically also connects the sensors to an electrical power source.

The wiring harness may be large and heavy, especially if a large number of sensors are distributed across a large area, for example if a large number of sensors are distributed across a fuel tank in a wing. The wiring harness may also be difficult to package within the wing so that it fits around other components in the wing and avoids electro-magnetic interference. The wiring harness may, therefore, be difficult to install and/or modify if it is subsequently necessary to reconfigure the sensor network and/or add new sensors, or replace wiring.

It is desirable to provide a sensor installation for an aircraft fuel system which addresses these problems. Similarly, it may be desirable to provide a sensor installation for any fuel tank or fuel system which reduces the weight of the sensor installation and reduces problems associated with wiring installations.

### SUMMARY OF THE INVENTION

A first aspect of the invention provides a sensor device for a fuel system, the sensor device comprising sensing means configured to sense a property of fuel within the fuel system, and transmitting means configured to wirelessly transmit a signal representative of the sensed fuel property to a remote receiver.

A second aspect of the invention provides a sensor system comprising one or more of the sensor devices.

A third aspect of the invention provides a fuel system comprising a sensor system according to the second aspect of the invention.

A fourth aspect of the invention provides an aircraft comprising a fuel system according to the third aspect of the invention.

A fifth aspect of the invention provides a sensor device comprising sensing means and a photovoltaic device having a photo-diode, wherein the photo-diode is operable to function as an antenna or part of an antenna for the device.

A sixth aspect of the invention provides a method for communicating with a plurality of sensor devices, the method comprising the steps of: consecutively transmitting request signals, each request signal identifying a respective one of the sensor devices; receiving the request signals at the respective sensor devices; and the sensor devices each consecutively transmitting a wireless signal when prompted by the request signal received.

A seventh aspect of the invention provides an aircraft having a fuel system including a sensor system comprising a wireless sensor device and a remote receiver on-board the aircraft, the sensor device comprising an energy storage device, sensing means configured to sense a property of fuel within the fuel system, and transmitting means configured to wirelessly transmit a signal representative of the sensed fuel property to the remote receiver.

An eighth aspect of the invention provides a method for communicating with a plurality of sensor devices on-board an aircraft, each sensor device comprising an energy storage device, the method comprising the steps of: consecutively transmitting request signals using a remote transmitter on-board the aircraft, each request signal identifying a respective one of the sensor devices; receiving the request signals at the respective sensor devices; and the sensor devices each transmitting a wireless signal when prompted by the request signal received.

The signal may be a coded radio signal or a microwave signal or an infrared signal, or any other type of signal which is suitable for wireless communication.

The signal transmitted by the transmitting means to the remote receiver may be communicated to a processing system, e.g. a core processing and input output module (CPIOM) or to a data collector, e.g. a remote data concentrator (RDC), which may communicate with the CPIOM over a data bus. The wireless communication between the sensor device and the remote receiver may eliminate the need for the sensor device to be wired into a wiring harness when installed in a fuel system. By eliminating the need to connect the sensor device to a wiring harness, the weight of the sensor installation may be reduced and the problems associated with wiring the sensor into a fuel system avoiding physical obstacles and sources of electro-magnetic interference may be reduced or eliminated. The invention may also increase ease of manufacture, assembly and maintenance by eliminating the need to install a signal wire leading to the sensor, or to connect and disconnect a signal wire during maintenance activities.

By providing a sensor system having a plurality of the sensor devices adapted to communicate wirelessly, the sensor system of the second aspect may eliminate the need for a wiring harness which would otherwise be required to connect a network of sensor devices to a processing system or data collector or remote power source in a conventional wired sensor system.

The sensor device may further comprise receiving means, and the sensor device may be configured to wirelessly transmit a signal to a remote receiver in response to a request received by the receiving means from a remote transmitter. The remote transmitter may transmit the request signal wirelessly to the receiving means. The remote transmitter may form part of the sensor system.

The sensor device may be configured to receive a request signal comprising an error check code, and the error check code may invalidate the signal so that the sensor device does not transmit a signal in response to the request signal if the request signal is corrupted, for example due to interference from an external source. The wireless signals transmitted by the sensor device may also contain an error check code configured to invalidate the signal if it becomes corrupted.

The transmitting means may have an operative active mode in which it is operable to wirelessly transmit a signal and an operative dormant mode in which it is operable to not wirelessly transmit a signal. By providing the transmitting means with an operative dormant mode in which it does not transmit a signal, the power consumption of the sensor device may be reduced. The receiving means may be operable to receive a request when the transmitting means is in its dormant mode.

The sensor device may further comprise an energy harvesting device. The energy harvesting device may be configured to provide the sensing means and the transmitting means with power, so that the sensing device does not need to be connected to another external power source. By providing the sensor device with an energy harvesting device, the need for wiring to connect the sensor device to another power source, for example a power cable or a wiring harness to a central power source or another remote power source, may be eliminated.

The sensor device may be self-powered, such that it does not require energisation by any power source associated with the aircraft or used for the aircraft but instead energises itself using energy from its surroundings, for example light energy, vibrations or changes in temperature.

A plurality of sensor devices may each have their own energy harvesting and storage devices. Alternatively, one or more of the sensor devices may share an energy harvesting and/or energy storage device with at least another sensor device.

The energy harvesting device may be a photovoltaic device. Alternatively, the energy harvesting device may be a vibration harvesting device or a micro fuel cell or a thermoelectric energy harvesting device or any other device suitable for harvesting energy.

The photovoltaic device may comprise a diode or diode array which is operable to function as an antenna or a part of an antenna for the device. The diode may be a photo-diode, and may be operable as an antenna or part of an antenna to receive a signal from a remote transmitter and/or to send a signal to a remote receiver.

The sensor device may further comprise an energy storage device. The energy storage device may be a rechargeable energy storage device which is connected to the energy harvesting device. The energy storage device may, for example, be a battery or a capacitor.

The sensor device may be configured to store an identifier. Preferably the identifier is a unique identifier. Preferably the identifier is reconfigurable. The identifier may be used to identify the sensor device when it transmits a signal. The identifier may, for example, be an RFID tag. Alternatively the identifier may be a bar code.

The sensing means may include a probe for projecting inside a fuel tank.

The sensor device may comprise mounting means for mounting to a fuel tank boundary. The mounting means may comprise, for example holes for receiving fasteners and/or an attachment bracket.

The sensor device may further comprise shielding means for electro-magnetically shielding the sensing means from the signal transmitted by the transmitting means. The shielding means may be arranged to reduce the amount of energy transmitted into a fuel system or fuel tank from the transmitting means.

The sensing means may be configured to determine one or more of: the quantity or temperature or density of fuel in a fuel tank or the amount of a contaminant in the fuel. The contaminant may be, for example, oxygen or water, or any other fuel contaminant which it is desirable to monitor.

The sensing means may generally be any type of sensor which may be used to determine a property of fuel within a fuel system, for example a vehicle fuel system or a stationary fuel tank. The sensor devices of the sixth aspect of the invention may comprise sensing means for measuring a property which is not a fuel property or a fuel system property. The sensing means of the sixth aspect may, for example, comprise a position sensor or a temperature sensor or a position sensor or any other type of sensor for use in any fuel system or non fuel system application.

The sensor device may comprise a plurality of sensing means. The plurality of sensing means may each be configured to sense the same property or alternatively to sense a plurality of different properties.

The sensor system of the second aspect may comprise one or more remote receivers configured to wirelessly receive a signal transmitted by the sensor device(s). The sensor device(s) may each be configured to transmit a wireless signal to one sensor device or to a plurality of sensor devices.

The sensor system may further comprise one or more remote transmitters configured to wirelessly transmit a request signal to the sensor device(s). Each request signal may be unique to each respective sensor device.

The sensor system may be able to detect the presence of one or more similar sensor systems and cooperate with the other sensor system(s) so that each of the sensor systems transmits requests to its sensor devices and transmits wireless signals from its sensor devices in its own time slot which does not overlap with the time slot(s) of the other sensor system(s).

The remote transmitter may be configured to wirelessly transmit a plurality of the request signals consecutively. When a plurality of request signals are transmitted consecutively, each request signal being unique to one of the respective sensor devices, a plurality of sensor devices may consecutively transmit wireless signals in response to their respective request signals. Each of the sensor devices may, therefore, transmit a wireless signal in turn in its own time slot, i.e. each wireless signal transmitted by one of the sensor devices in response to a request signal is not concurrent with any other wireless signal transmitted by another one of the sensor devices in response to another request signal. The sensor devices may therefore transmit their wireless signals in synchronised, pulsed responses. By requesting each sensor device to transmit a signal individually in its own time slot, interference between signals from the different sensor devices may be reduced.

The remote transmitter may request a wireless signal from a particular sensor device only when a sensed property from that sensor device is desired, for example after a pre-determined time period or in response to a particular event. The transmitting means may, therefore, only enter its operative active mode when a response is requested from the sensor, so that the power consumption of the sensor device is minimised. The response may be a short pulsed signal.

The unique wireless request signals may be unique within a sensor system. Additionally the unique wireless request signals may be unique across a plurality of sensor systems. By making the wireless request signals unique across a plurality of sensor systems, the interference between the systems may be reduced.

The fuel system of the third aspect may further comprise a fuel tank. The sensing means of the sensor device(s) may extend into the fuel tank, and the sensor device(s) may be adapted to be removed from outside the fuel tank, preferably without requiring access inside the fuel tank. The sensor device(s) may, therefore, be quickly and easily removed from the fuel tank without disassembling the fuel tank or performing any operations on the inside of the fuel tank.

The sensor system may be arranged such that wireless signal paths between nodes of the sensor system are substantially external to the fuel system. By providing a path which is substantially external to the fuel system, the amount of energy absorbed by the fuel system is reduced.

The aircraft of the fourth aspect may further comprise a wing having an upper structural cover, and the sensor device(s) may be attached to the upper wing cover. The sensor devices may therefore be top-mounted with respect to the wing. The sensor device(s) may be adapted to be removed through the upper wing cover externally without requiring access to the inside of the wing. By arranging the sensor device(s) on the upper wing cover, the sensor device(s) may be removed when there is fuel in the fuel tank without requiring the tank to be drained first.

In the method of the sixth aspect, the request signals may include an identifier which is unique to the respective sensor devices.

The wireless signals transmitted by the sensor devices of the eighth aspect may be representative of sensed properties determined by the sensor devices. The wireless signals transmitted by the sensor devices may be received by one or more remote receivers on-board the aircraft.

Each wireless signal transmitted in response to one of the request signals may not be concurrent with any other wireless signal transmitted in response to another request signal. Interference between wireless signals transmitted from each of the respective sensor devices may, therefore, be minimised or avoided entirely. Transmitting the consecutive request signals may be timed to avoid signal interference.

It will be appreciated that the features of the various aspects of the invention may be combined with those of other aspects of the invention.

### BRIEF DESCRIPTION OF THE DRAWINGS

Embodiments of the invention will now be described with reference to the accompanying drawings, in which:
Figure 1 illustrates a plan view of an aircraft showing schematically the locations of wing mounted sensor devices;
Figure 2 illustrates a cross section through a wing of an aircraft;
Figure 3 illustrates a sensor device; and
Figure 4 illustrates a typical master/slave data exchange sequence.

### DETAILED DESCRIPTION OF EMBODIMENT(S)

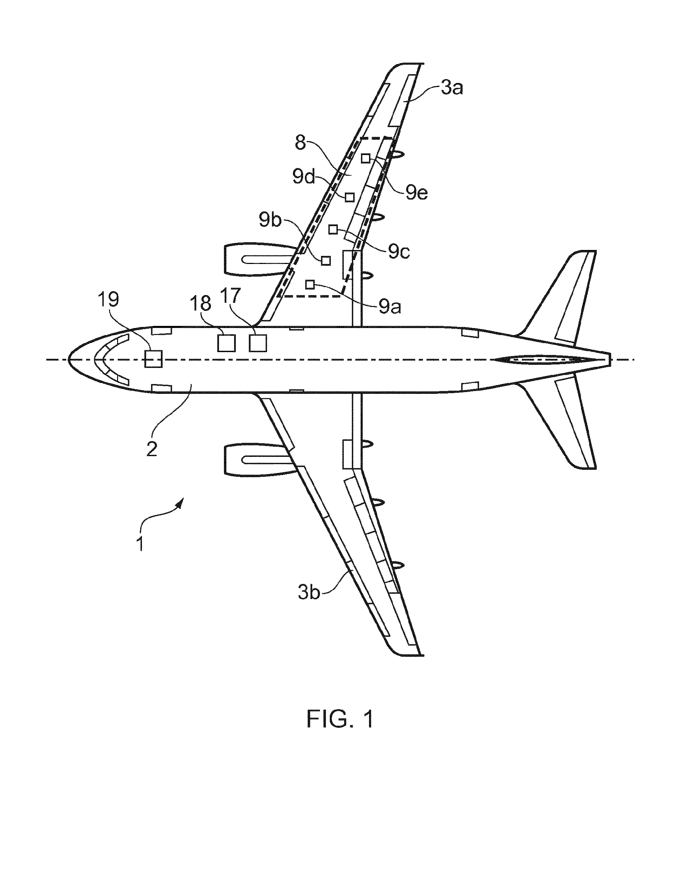

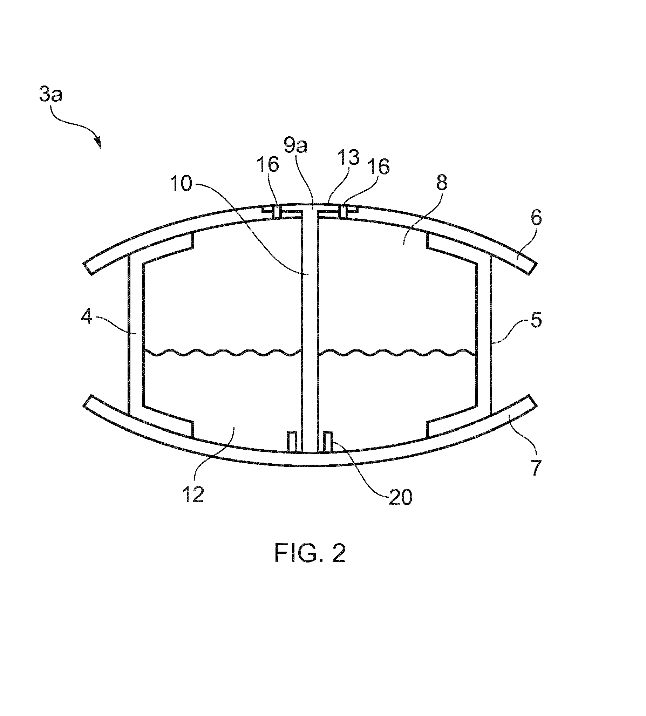

Figure 1 illustrates an aircraft 1 having a fuselage 2 and wings 3a, 3b. The wing 3a has a wing box, shown in figure 2, comprising front and rear spars 4, 5 and upper and lower covers 6, 7. The wing 3a defines an integral fuel tank 8, the location of which is indicated by the broken line in figure 1. The front and rear spars 4, 5 form the front and rear walls of the fuel tank 8 and the upper and lower covers 6, 7 form the upper and lower walls of the fuel tank 8.

The fuel tank is provided with a plurality of sensor devices 9a-9e which are distributed across its extent, the locations of which are indicated in figure 1. It should be noted that the fuel tank 8 has other sensor devices which are not shown for clarity. The fuel tank may, for example, be provided with e.g. 1000 or more sensors associated with various sensor devices.

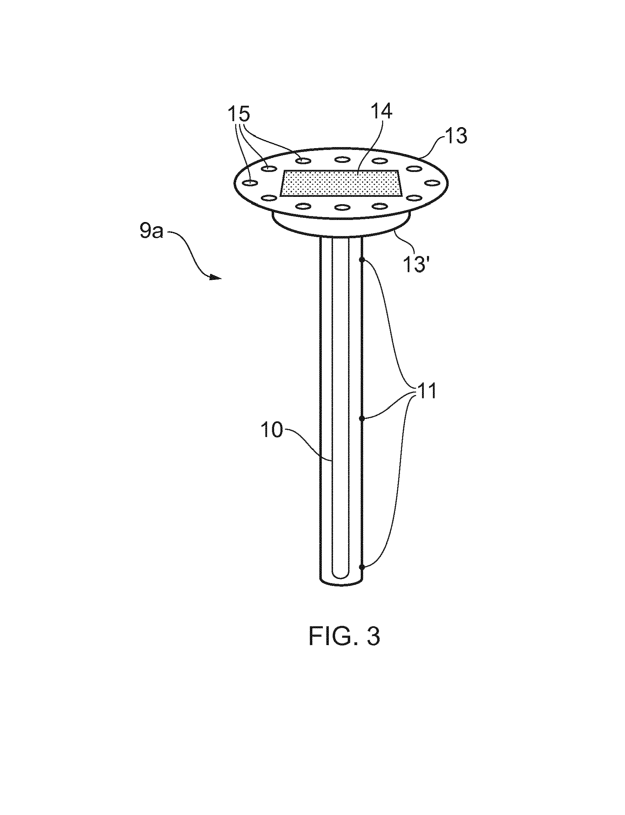

Sensor device 9a, shown in figure 3, comprises a fuel measurement probe 10 and a plurality of fuel temperature sensors 11 which are mounted on the fuel measurement probe. The fuel measurement probe 10 is adapted to sense the level of fuel 12 within the fuel tank 8 and extends substantially between the upper cover and the lower covers 6, 7, as indicated in figure 2 which illustrates a cross section through the wing 3a and the sensor device 9a. The fuel temperature sensors 11 are adapted to sense the temperature of fuel 12 within the fuel tank 8. Each of the sensor devices 9a-9e is adapted to measure one or more fuel property, for example one or more of: fuel level or temperature or density or amount of a contaminant, e.g. water, in the fuel 12. The sensor devices may measure an amount of a contaminant for example by measuring the permittivity and/or resistivity of the fuel.

Sensor device 9a further comprises a flange portion 13 which houses a photovoltaic device 14, a rechargeable battery (not shown) and an electronics unit (not shown) in a flange canister 13' below the photovoltaic device. The rechargeable battery and electronics unit may be removed from the flange canister and replaced during maintenance activities without requiring the removal of the sensor device 9a from the wing 3a.

The flange portion 13 has a plurality of holes 15 arranged in a ring around the flange portion extending through its thickness. The holes 15 receive mechanical screw fasteners 16 which attach the flange portion to the upper wing cover 6. The upper surface of the flange portion 13 is substantially flush with the upper surface of the upper wing cover 6 to minimise drag. The flange portion is adapted to be flexible to accommodate changes in the wing shape, for example changes due to aero-elastic deformation. By positioning a photovoltaic device on the upper cover of a wing, the photovoltaic device may be located in a good position for receiving direct sunlight.

The lower wing cover 7 has a funnel guide 20 on its upper surface which receives the lower end of the probe 10. The funnel guide facilitates correct insertion of the sensor device when it is mounted and improves location of the probe in service.

The sensor device 9a may be removed through the upper wing cover by removing the mechanical fasteners 16 and lifting the sensor device out of the wing 3a. By arranging the sensor device 9a to be removed through the upper wing cover 6, the sensor device 9a may be removed when there is fuel 12 in the fuel tank 8 without requiring the fuel tank to be drained first and without any risk of the fuel leaking out of the fuel tank. The sensor device 9a is adapted to be removed without requiring access to the inside of the fuel tank 8 to increase ease of maintenance activities.

Some of the other sensor devices are arranged similarly to sensor device 9a and have attachment flanges for attachment to the upper wing cover 6. However, other sensor devices associated with the fuel tank 8 may be attached differently, and may be attached to other parts of the aircraft, for example to the front or rear spars 4, 5 or to the lower wing cover 7.

The photovoltaic device 14 is adapted to generate electrical energy when solar radiation is incident upon it. The rechargeable battery is adapted to store energy generated by the photovoltaic device 14. The photovoltaic device 14 and rechargeable battery form a power source for the sensor device 9a, which supply electrical power to the measurement probe 10, the temperature sensors 11 and the electronics unit. The rechargeable battery is adapted to be able to supply power to the sensor device 9a for a prolonged period, e.g. up to 48 hours (from fully charged) so that the sensor device can continue to function during time periods in which the photovoltaic device 14 does not produce enough energy to power the sensor device, for example when there is insufficient solar radiation incident upon the photovoltaic device at night or when the aircraft is in temporary hangar storage.

Some of the other sensor devices also comprise photovoltaic devices and batteries in their flange portions. However, other sensor devices, for example those which are not attached to the upper wing cover 6, may have other types of energy harvesting devices, for example vibration harvesting devices.

Each of the sensor devices 9a-9e has an electronics unit. The electronics unit of each sensor device 9a-9e comprises a transmitter, a receiver, a microprocessor, memory and sensor conditioning electronics. The transmitter is configured to wirelessly transmit signals representative of the sensed fuel properties to a remote transceiver 17 which is located in the fuselage 2, as shown in figure 1. The transmitter uses a photodiode or array of photodiodes as an antenna or a part of an antenna to broadcast and receive the signals. The dimensions of the diode or diode array are matched to the required antenna dimensions for the frequency of transmission and reception with additional lengths of connecting wires where appropriate. The diode(s) are appropriately direction polarised.

The signals transmitted by the transmitter are coded radio frequency signals which are received by the remote transceiver. The transmitter has an operative active mode in which it transmits a signal and an operative dormant mode or passive mode in which it does not transmit a signal.

The remote transceiver 17 communicates the fuel properties to a data collector 18 which sends data relating to the aircraft fuel system to a data processing system 19. By enabling the sensor devices 9a-9e to wirelessly communicate sensed fuel properties to a remote transceiver 17, the sensor devices are able to provide data relating to the fuel 12 in the fuel tank 8 to the data processing system 19 without being electrically connected to a data collector or to any other external device by wires. It is therefore possible to integrate the sensor devices 9a-9e without requiring a wiring harness.

The wireless sensor devices 9a-9e reduce the need for wiring in the aircraft fuel system and eliminate the need for a wiring harness to connect the sensors to the processing system 19. By eliminating the wiring harness, the weight of the sensor installation is significantly reduced, and the problems associated with routing of sensor wires through the aircraft wing 3a avoiding physical obstacles and sources of interference are reduced. The space available for receiving other systems within the wing 3a, for example power harnesses and activation cables which would otherwise need to be spaced apart from the sensor wires or wiring harness, is also increased. These advantages are particularly important for sensors located in an aircraft wing where packaging of wires can be problematic. The wireless sensor devices 9a-9e may be used to replace at least some, and possibly all, conventional wired fuel system sensor devices. It may be desirable to replace only some of the conventional wired fuel system sensor devices (e.g. in difficult to access regions, or where long wiring runs may be required) and to retain some wired sensor devices (e.g. in highly critical locations).

The wireless sensor devices 9a-9e also increase the ease of manufacture, assembly and maintenance of the sensor network for the fuel tank 8 because there is no need to install wires leading to sensors or to connect and disconnect signal wires and/or power wires during maintenance activities. The reliability of the sensor network is also improved because faults due to failure of connecting wires and connectors are substantially eliminated.

The transmitter is also configured to transmit a signal representative of the state of the rechargeable battery to the remote transceiver 17 so that the processing unit can determine whether the photovoltaic device 14 is functioning correctly and can determine if the battery state is low so that the sensor device 9a might become unable to function due to a lack of power. If the battery state is low then the data processing system 19 may respond by requesting a signal from the sensor device 9a less frequently so that energy consumption is reduced and the battery life is prolonged. If the battery state is very low then the sensor device may enter a "sleep mode" in which no signal is transmitted to prevent full discharge. The sensor device may then be reactivated and recommence transmitting signals when recharge is detected, for example if the aircraft 1 is moved back into sunlight after a prolonged period during which the photovoltaic device was not exposed to sufficient light.

The transmitter is also configured to transmit an identifier when it transmits a signal. Each of the sensor devices has a unique identifier in the form of a radio-frequency identification tag (RFID tag) and a reader which reads the RFID tag. The RFID tags are set when the sensor devices 9a-9e are installed on the wing 3a, and the RFID codes are uploaded to a table in the aircraft's fuel management control module so that the individual sensor devices can be readily identified. Each sensor device then uses its reader to read its RFID tag and stores the identifier in its memory so that each sensor device can recall its own unique identifier. The unique identifiers of the sensor devices 9a-9e are reconfigurable so that the identifiers may be reprogrammed as required, for example if the sensor configuration is changed or if new sensor devices are added to the fuel tank 8.

The unique identifiers contain information relating to the unique location of the sensor device on the aircraft and the part serial number of the sensor device. The unique location identifiers are received by the remote transceiver 17 so that the processing system 19 can associate the properties sensed by the fuel sensors with the sensor device which supplied that signal. The unique RFID tags may also help with maintenance operations because they can be used to help locate a particular sensor or to identify a sensor which is being inspected, for example by using a reader to read the RFID tag to ascertain or confirm which sensor is being inspected.

The receivers of the sensor devices 9a-9e are configured to receive request signals from the remote transceiver 17. The sensor devices 9a-9e are configured to respond to the request signals, and the microprocessors, having detected a request signal, respond by ordering transmission of a signal representative of the sensed fuel properties to the remote transceiver. The sensor devices are activated to take measurements shortly after the transmission of the previous signal, and the memory is configured to store data relating to the sensed properties of the fuel 12 in the fuel tank 8 until the next signal is transmitted. The sensor devices therefore operate by first receiving a request signal, then transmitting a signal representative of a previously sensed fuel property, and finally measuring a fuel property which may be communicated following the next request signal. The three stages of reception, transmission and measurement do not, therefore, take place at the same time, and so power spikes are reduced.

The sensor devices 9a-9e are configured to transmit a signal only when a request is received from the remote transceiver 17. The transmitters of the sensor devices 9a-9e remain in the operative dormant mode when they are not transmitting a signal. However, the receivers can still receive a signal from the remote transceiver 17 when the transmitting means are in the operative dormant mode. By transmitting data relating to fuel system properties only when the data is required by the processing unit, the amount of time for which the transmitting means transmits a signal may be reduced. By reducing the amount of time which the transmitter is transmitting signals (in the operative active mode), the overall power consumption of the sensor devices 9a-9e is reduced, so that the requirements placed on the power sources is reduced. The sensor devices take measurements in response to receiving a request signal and remain in a dormant state at other times so the power consumption of the sensor devices is further reduced. The signals transmitted by the sensor devices 9a-9e have an energy of approximately 50µJ, and are within the 200µJ limit set out under AC25/981C. Other limits, higher or lower, may be applicable according to local rules and regulations. The sensor devices can, therefore, function with comparatively small and lightweight power sources and without transmitting higher than allowable energy signals near or into the fuel tank 8.

The remote transceiver 17 sends a unique request signal to each of the sensor devices 9a-9e consecutively, and the sensor devices respond by consecutively transmitting signals representative of the sensed fuel properties in response to their own unique request signals. The sensor devices 9a-9e have a refresh time of 1 second, so that the request and response events for the plurality of sensor devices occurs consecutively and in a predetermined sequence once every second. By requesting signals individually from the sensor devices 9a-9e so that each of the sensor devices transmits a signal in its own time slot, the possibility of interference between the signals from the different sensor devices 9a-9e is reduced or negated. Each time slot has a duration of 0.001 seconds. By adapting each sensor device to transmit a signal for a short time period within the refresh period, the energy consumption of the sensor device may be minimised.

Each of the unique request signals is unique within the sensor system to reduce interference between the individual sensor devices 9a-9e. Additionally, the unique request signals are unique across a fleet of aircraft to minimise or negate the possibility of interference between aircraft which are parked adjacent each other.

When the aircraft 1 is co-parked adjacent another aircraft having a comparable sensor system, the two aircraft each detect the presence of the other and the sensor systems of the respective aircraft cooperate so that the two sensor systems each operate in a separate time slot. The refresh time for each of the two sensor systems increases to 2 seconds, and the sensor systems of the respective aircraft alternate between 1 second of operation (as described above) while the other aircraft does not transmit request signals or response signals, followed by one second of inactivity in which no request signals are transmitted. Interference between the sensor systems of the two aircraft is therefore reduced. If three aircraft are co-parked then the refresh time for each sensor system may increase to 3 seconds and the three aircraft may cooperate similarly to eliminate interference between their respective sensor systems.

The path of signals sent between the sensor device 9a and the remote transceiver 17 is substantially external to the fuel tank 8, and the flange portion 13 acts as an electromagnetic shield to reduce the amount of transmitted signal energy from the sensor device 9a which passes into the fuel tank. Additionally, the walls of the fuel tank 8 act as a Faraday cage grounded to the wing structure, so that the energy of the signals is substantially prevented from entering the interior of the fuel tank.

The path of signals sent between the sensor device 9a and the remote transceiver 17 is substantially through free air. The proportion of the signal energy which is absorbed between the sensor device 9a and the remote transceiver 17 is, therefore, minimised. In this way the energy required to successfully transmit a wireless signal between the sensor device 9a and the remote transceiver 17, and therefore the energy consumption of the sensor device, is reduced.

Each request signal transmitted by the remote transceiver 17 and each response signal transmitted by one of the sensor devices 9a-9e has a verification error check code, for example a Reed Solomon code, which is transmitted as part of the signal. The error check code invalidates the signal so that the signal will be ignored if any data in the signal is corrupted, for example due to interference from an external source such as another aircraft when the aircraft 1 is on the ground. If a request and response event for a particular sensor device is disrupted in this way, the last successfully transmitted signal from that sensor device is retained until the interference subsides and request and response events can recommence.

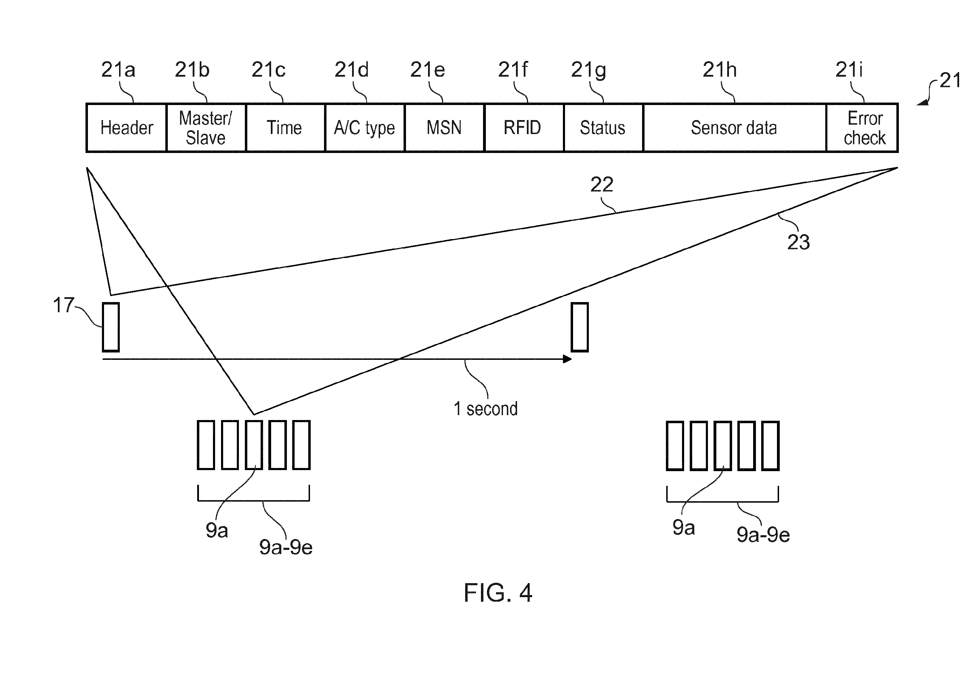

Figure 4 shown a typical master/slave data exchange sequence between the remote transmitter 17 (the master) and one of the sensor devices 9a-9e (the slave), as recorded by the processing system 19. The form of the request and response signals is indicated at line 21, each signal including the following data: a manufacturer's header 21a, a master/slave identifier 21b, a time record 21c, an aircraft model identifier 21d, the aircraft Manufacturer's Serial Number (MSN) 21e, a sensor device RFID (or other identifier) 21f, a sensor device status entry (ie battery state) 21g, a sensor data entry (ie sensed fuel property or properties) 21h and an error check code 21i.

When the remote transmitter 17 transmits a request signal 22, the master/slave identifier identifies the signal as a request signal being sent from the transceiver to the sensor devices. The MSN, which is unique within a fleet of aircraft, identifies the signal as a signal from the aircraft 1 and not, for example, a signal from another aircraft. The sensor device RFID identifies the target sensor device ie the sensor device from which a response is desired, for example sensor 9a as shown in figure 4. The sensor device status entry and sensor data entry are blank because these values are to be filled in by the sensor device.

If the request signal 22 is received by the target sensor device 9a (as identified by the RFID part of the request signal) and the error check code in the request signal does not cause the request to be ignored, the sensor device 9a recognises itself as the target sensor device and transmits a signal 23 in response to the request signal. The sensor device status and sensor data entries are completed in the returned signal 23, and indicate the most recently determined values for the battery state and the sensed fuel properties. The RFID of the sensor device is also included in the returned signal 23 to identify the sensor device 9a as the sensor device which transmitted the returned signal. Each of the sensor devices 9a-9e which are in communication with the remote transceiver 17 communicate using a similar data exchange sequence, and the process is repeated for each sensor device once per refresh period ie once every second.

In another embodiment, a single request signal may be transmitted by the remote transmitter which is received by more than one of the sensor devices or all of the sensor devices, and the sensor devices may each respond to the request signal in their own separate time intervals. For example, a plurality of sensor devices may receive a single request signal and may each respond consecutively so that a first one of the sensor devices transmits a wireless signal in response to the request in a first time slot, and then a second one of the sensor devices transmits a wireless signal in response to the request in a second time slot and so on.

In another embodiment, the remote transceiver 17 may be replaced with one or more remote transmitters for sending request signals to the sensor devices and one or more remote receivers for receiving signals from the sensor devices.

In another embodiment, the request signals may not be transmitted consecutively, but some of the request signals may be transmitted at the same time. In another embodiment, the time slot in which each of the sensor devices transmits a signal may have a duration which is different to 0.001 seconds.

In another embodiment, at least one of the sensor devices 9a-9e may share an energy harvesting device and/or an energy storage device with at least one of the other sensor devices 9a-9e.

In another embodiment, the rechargeable battery may be replaced with a super capacitor, or another suitable energy storage device.

In another embodiment, the energy storage device may be adapted to be able to supply power to the sensor device for a period other than 48 hours (from fully charged), for example longer than 48 hours or 24 hours or an hour, without energy being generated or delivered by the energy harvesting device.

In an another embodiment, the battery and/or electronics and/or energy harvesting device may be in a location remote from the sensing means. For example, a sensor device may have a sensor or sensors which extend into a fuel tank and a battery and energy harvesting device which are located outside the fuel tank, for example on the outer wall of one of the spars 4, 5. The battery and energy harvesting device may be connected to the sensor device by wires.

In another embodiment, the sensor devices may not use photo diodes as antennas to receive and broadcast signals but may instead all use conventional antenna designs known from the prior art.

In another embodiment, the reader of each sensor device may be used to read the RFID tag each time information about the sensor device's identifier is required instead of reading the RFID tag once and then storing the identifier in the memory. In another embodiment, each sensor device may not have a reader but may instead be programmed to remember its own identifier without having to read it using a reader. In another embodiment, each sensor device may comprise a bar code which may perform the same function as the RFID tag described above. In another embodiment, each sensor device may have an identifier which is not in the form of an RFID tag or a bar code but is any other known type of identifier which may be read or stored in the memory.

In another embodiment, the sensor devices may be located in different locations to those shown in figure 1. In another embodiment, the fuel tank 8 may be located in a different location in an aircraft such as aircraft 1, for example in wing 3b or in the fuselage or in a vertical or horizontal stabiliser. In another embodiment, the sensor devices may be distributed across more than one fuel tank, or in any other part of a fuel system. For example, sensor devices may be distributed across at least one fuel tank in wing 3a and at least one fuel tank in wing 3b.

In another embodiment, a sensor device or sensor devices may be installed in a fuel system which is not an aircraft fuel system, for example a stationary fuel storage tank, or in a fuel system for another type of vehicle, for example a car or a train or a ship. In any of these alternative embodiments, the wireless sensor devices may provide similar benefits in terms of weight, packaging, manufacture and maintenance as described above for the sensor devices as installed on the aircraft 1.

Any feature or combination of features from any of the embodiments described above may be appropriately combined with any feature or combination of features from any other embodiment or embodiments.

Although the invention has been described above with reference to one or more preferred embodiments, it will be appreciated that various changes or modifications may be made without departing from the scope of the invention as defined in the appended claims.

## Claims

1. An aircraft having a fuel system including a sensor system comprising a wireless sensor device and a remote receiver on-board the aircraft, the sensor device comprising an energy storage device, sensing means configured to sense a property of fuel within the fuel system, and transmitting means configured to wirelessly transmit a signal representative of the sensed fuel property to the remote receiver.

2. An aircraft according to claim 1, wherein the sensor system comprises a remote transmitter and the sensor device further comprises receiving means, wherein the transmitting means is configured to wirelessly transmit a signal to the remote receiver in response to a request received by the receiving means from a remote transmitter.

3. An aircraft according to any preceding claim, wherein the transmitting means has an operative active mode in which it is operable to wirelessly transmit a signal and an operative dormant mode in which it is operable to not wirelessly transmit a signal.

4. An aircraft according to any preceding claim, wherein the sensor device further comprises an energy harvesting device.

5. An aircraft according to claim 4, wherein the energy harvesting device is a photovoltaic device.

6. An aircraft according to claim 5, wherein the photovoltaic device comprises a diode or diode array which is operable to function as an antenna or part of an antenna for the device.

7. An aircraft according to any preceding claim, wherein the sensor device is configured to store an identifier.

8. An aircraft according to any preceding claim, wherein the sensing means includes a probe for projecting inside a fuel tank.

9. An aircraft according to any preceding claim, wherein the sensor device further comprises mounting means for mounting to a fuel tank boundary.

10. An aircraft according to any preceding claim, wherein the sensor device further comprises shielding means for electro-magnetically shielding the sensing means from the signal transmitted by the transmitting means.

11. An aircraft according to any preceding claim, wherein the sensing means is configured to sense one or more of: a level or temperature or density of fuel in a fuel tank, or an amount of a contaminant in a volume of fuel in a fuel tank.

12. An aircraft according to any preceding claim, wherein the sensor device includes a plurality of the sensing means.

13. An aircraft according to any preceding claim comprising one or more of the sensor devices.

14. An aircraft according to claim 13 when dependent on claim 2, wherein the remote transmitter is configured to wirelessly transmit a request signal to the sensor device(s), wherein each request signal is unique to each respective sensor device.

15. An aircraft according to claim 13 when dependent on claim 2 or according to claim 14, wherein the remote transmitter is configured to wirelessly transmit a plurality of the request signals consecutively.

16. An aircraft according to any preceding claim, wherein the request signals are timed such that each of the sensor devices transmits a wireless signal in its own time slot which is not concurrent with that of any other sensor device.

17. An aircraft according to any preceding claim, further comprising a fuel tank, wherein the sensing means of the sensor device(s) extends into the fuel tank, and wherein the sensor device(s) are adapted to be removed from outside the fuel tank, preferably without requiring access inside the fuel tank.

18. An aircraft according to any preceding claim, wherein sensor system is arranged such that wireless signal paths between nodes of the sensor system are substantially external to the fuel system.

19. An aircraft according to any preceding claim, further comprising a wing having an upper structural cover, wherein the sensor device(s) are attached to the upper wing cover.

20. An aircraft according to claim 19, wherein the sensor device(s) are adapted to be removed through the wing cover without requiring access to the inside of the wing.

21. A method for communicating with a plurality of sensor devices on-board an aircraft, each sensor device comprising an energy storage device, the method comprising the steps of: consecutively transmitting request signals using a remote transmitter on-board the aircraft, each request signal identifying a respective one of the sensor devices; receiving the request signals at the respective sensor devices; and the sensor devices each transmitting a wireless signal when prompted by the request signal received.

22. A method according to claim 21, wherein the request signals include an identifier which is unique to the respective sensor devices.

23. A method according to claim 21 or 22, wherein transmitting the consecutive request signals is timed to avoid signal interference.

24. A method according to any of claims 21 to 23, wherein the sensor devices respond to their respective request signals by transmitting synchronised, non-concurrent wireless signals.

25. A method according to any of claims 21 to 24, wherein the wireless signals transmitted by the sensor devices are representative of sensed properties.

26. A method according to any of claims 21 to 24, wherein the wireless signals transmitted by the sensor devices are received by one or more remote receivers on-board the aircraft.
